# EUROPEAN PATENT APPLICATION

(11) **EP 2 567 697 A1**
(43) Date of publication of application: **13.03.2013**
(21) Application number: 11382291.0
(22) Date of filing: 12.09.2011
(51) Int. Cl.: A61K 31/196, A61K 31/728, A61P 17/00

(54) **Topical pharmaceutical compositions**

(71) Applicant: Almirall, S.A., 08022 Barcelona (ES)
(72) Inventor: Fielhauer, Sabine, 21465 Reinbek (DE); Trommer, Hagen, 21465 Reinbek (DE); Strahinjic, Ivana, 21465 Reinbek (DE); Mallwitz, Henning, 21465 Reinbek (DE); Willers, Christoph, 21465 Reinbek (DE)
(74) Representative: Elzaburu Marquez, Alberto

(57) **Abstract**

Topical pharmaceutical compositions are described comprising, based on the total weight of the composition:
(a) 4.0-5.5 wt.% of diclofenac sodium;
(b) 1.5-3.0 wt.% of hyaluronic acid, a salt thereof or mixtures thereof;
(c) 50-60 wt.% of water; and
(d) 30-40 wt.% of a carrier or vehicle other than water.

Said compositions are stable and can be safely and easily applied over large surface areas of the skin in an acceptable way by the general patient population for the treatment or prevention of some skin disorders or diseases.

## Description

### FIELD OF THE INVENTION

The present invention relates to topical pharmaceutical compositions comprising (a) diclofenac sodium and (b) hyaluronic acid, a salt thereof or mixtures thereof. The invention further relates to methods of treatment of some skin diseases or disorders by administering the compositions.

### BACKGROUND OF THE INVENTION

The skin is the largest organ of the body and comprises the epidermis and dermis. The skin is composed of different tissue compartments that interconnect and interact in different ways.

Skin cancer is the most common form of cancer in men. Basically the scientific community differentiates between malignant melanoma and non-melanoma skin cancer (NMSC). Usually basal cell carcinoma and squamous cell carcinoma are classified into the group of NMSC.

Basal cell carcinoma (BCC) is a malignant neoplasm derived from non-keratinizing cells that originate in the basal layer of the epidermis. If basal cell carcinoma is not treated it will invade from the skin into local structures and could induce substantial tissue damage. Metastases are extremely rare. BCC is the most common cancer in human.

Squamous cell carcinomas (SCC) of the skin are malignant neoplasms derived from suprabasal epidermal keratinocytes. SCC probably derives from precursor lesions of actinic keatoses (AK) and Bowen's disease. Cutaneous SCC represents a wired range of diseases from easy to manage superficial cancers to highly infiltrative tumors. Especially the later SCCs develop metastases in many cases that can result in death.

Actinic keratoses are cutaneous neoplasms consisting of proliferations of cytologically aberrant epidermal keratinocytes that develop in response to prolonged UV exposure. AKs have the potential to develop into SCCs. Several authors classify AKs as malignant neoplasms because the lesions are intraepidermal SCCs in evolution. Therefore AK can be seen as the initial lesion of a disease continuum which may evolve into SCC. Not all AKs necessarily develop into SCCs. AK can also be regarded as a predictor for the probability of a patient developing other forms of skin cancer including malignant melanoma. Treatment of AK is therefore an important action to prevent other forms of skin cancer.

Current treatments for AK consist of physical and pharmacological therapies and include (Guidelines for the management of AKs, D. de Berker et al., British Journal of Dermatology, 2007, 156, pp 222-230):
Physical treatments of AK include: Cryosurgery using liquid nitrogen, curettage, laser surgery and conventional surgery.

The pharmacologic therapies include:
- Photodynamic therapy, which involves applying a photosensitizing agent to each actinic keratosis, followed by exposure to light of a specific wavelength;
- Topical therapies including 5-fluorouracil creams (Effudex®, Carac®, Fluoroplex®, Actikerall®, etc.), imiquimod 5% cream (Aldara®), diclofenac sodium 3% gel (Solaraze ®). Non-approved topical treatments for AK include topical retinoids, trichloroacetic acid and others.

In the field of topical therapies for treating skin diseases or disorders, particularly in the treatment of actinic keratosis, there exists a need for compositions which a shorter duration of therapy and/or higher clearance of skin lesions.

Improving the current topical therapies by increasing the concentration of the active ingredient is not always possible because the concentration of the excipients of the composition need also to be modified, resulting in changes in the cosmetic properties of the new composition. These changes may affect the general acceptance by the patients and thereby influence patients' adherence to the treatment.

U.S. Patents Nos. 5639738, 5792753, 5852002, 5914322, 5929048 and 5985850, teach the use of topical compositions comprising 1-5 wt.% of a non-steroidal anti-inflammatory drug (NSAID), and hyaluronic acid or a pharmaceutically acceptable salt thereof, said hyaluronic acid having a molecular weight ranging between 150,000 to 750,000 Daltons, for the treatment of various skin disorders, including actinic keratosis. Said patents describe, for example, 1 wt.% diclofenac sodium, 3 wt.% diclofenac sodium, 5 wt.% ibuprofen, 2 wt.% piroxicam or 1 wt%. banamine compositions. TR200000050 discloses a dermatological preparation composition, which contains 1-10 wt.% of diclofenac sodium and 1-10 wt.% of hyaluronic acid. However no further information is provided on excipients or other ingredients in the composition.

WO03/105821 describes pharmaceutical compositions comprising 0.001-5 wt.% triamcinolone acetonide and 0.1-10 wt.% diclofenac sodium, as the active ingredients, for reliefing of pain and for the treatment of acute or chronic erosions in oral mucous membrane such as aphtous stomatitis, stomatitis (including denture stomatitis), lichen planus related to oral mucous membrane, painful lesions caused by the prosthesis. It is described a composition containg diclofenac sodium and triamcinolone acetonide as active agents, and benzyl alcohol, methoxy polyethylene glycol, water and hyaluronic acid as a gel former agent. However, no further information is provided on the concentration of hyaluronic acid or on other excipients.

Surprisingly, it has been found that topical pharmaceutical compositions comprising higher amounts of diclofenac sodium than the current therapies, exhibit similar rheological properties to these current therapies, without affecting the gel forming properties of hyaluronic acid (or salt thereof). Therefore, said topical pharmaceutical compositions exhibit a shorter duration of therapy and/or higher clearance of skin lesions, while keeping easy handling, easy spreadability and quick absorption by the skin.

### SUMMARY OF THE INVENTION

The present application refers to topical pharmaceutical compositions comprising, based on the total weight of the composition:
(a) 4.0-5.5 wt.% of diclofenac sodium;
(b) 1.5-3.0 wt.% of hyaluronic acid, a salt thereof or mixtures thereof;
(c) 50-60 wt.% of water; and
(d) 30-40 wt.% of a carrier or vehicle other than water.

Said compositions are stable and exhibit comparable rheological properties to diclofenac sodium compositions available in the market and exhibit a shorter duration of therapy and/or higher clearance of skin lesions. Therefore, said combinations are useful in the treatment of skin diseases or disorders.

The invention further relates to a composition as defined above for use in the treatment or prevention of skin disorders or diseases selected from a) epidermal and follicular tumors (such as basal cell carcinoma (BCC), squamous cell carcinoma (SCC), actinic keratosis (AK), Bowen's disease, erythroplasia of Queyrat, leukoplakia, oral florid papillomatosis, verrucous carcinoma or keratoacanthoma); b) sebaceous gland tumors (such as sebaceous carcinoma); c) sweat gland tumors (such as mammary Paget's disease, extramammary Paget's disease, eccrine porocarcinoma, microcystic adnexal carcinoma (MAC) or mucinous carcinoma of the skin); d) nervous system tumors (such as Merkel cell carcinoma (MCC)); e) mesenchymal tumors (such as dermatofibrosarcoma protuberans (DFSP), malignant fibrous histiocytoma (MFH), atypical fibroxanthoma, epithelioid sarcoma, synovial sarcoma, liposarcoma, angiosarcoma, Kaposi's sarcoma, cutaneous lymphoma or multiple myeloma); f) metastatic carcinoma of the skin; g) skin melanoma; h) lamellar ichthyosis; i) acne; j) hirsutism; k) androgenetic alopecia; l) pruritus (itch); or m) scar.

The invention further relates to the use of a composition as defined above for the manufacture of a medicament for the treatment or prevention of skin disorders or diseases as defined above.

The invention further relates to a method for treating a subject afflicted with skin disorders or diseases as defined above, which comprises applying to the affected area of skin of said subject an effective amount of a topical pharmaceutical composition as defined above.

### DETAILED DESCRIPTION OF THE INVENTION

### Diclofenac sodium

Diclofenac sodium (sodium [2-(2,6-dichloroanilino)phenyl]acetate; CAS RN 15307-79-6) is a non-steroid anti-inflammatory drug having the empirical formula C₁₄H₁₀Cl₂NNaO₂, and a molecular weight of 318.1 g/mol.
It is a white to slightly yellowish hygroscopic crystalline powder, sparingly soluble in water; soluble in alcohol; slightly soluble in acetone; practically insoluble in ether; and freely soluble in methyl alcohol (see Martindale - The complete drug reference, 32nd edition, 1999, pages 31-33).

### Hyaluronic acid (or salts thereof)

Hyaluronic acid (hyaluronan; CAS RN 9004-61-9) or sodium hyaluronate (CAS RN 9067-32-7) is a naturally occurring polyanionic, polysaccharide that consists of repeating disaccharide units of N-acetyl-D-glucosamine and β-glucoronic acid. Typically, hyaluronic acid refers to hyaluronic acid, or salts thereof or mixtures thereof. Preferred forms of hyaluronic acid according to the invention include hyaluronic acid, sodium hyaluronate or mixtures thereof. In a preferred embodiment, the hyaluronic acid is in the form of sodium hyaluronate.
Typically, the average molecular weight of hyaluronic acid or sodium hyaluronate ranges from 150 kD to 900 kD. Preferably, the average molecular weight of hyaluronic acid or sodium hyaluronate ranges from 300 kD to 850 kD, more preferably from 600 kD to 800 kD, even more preferably from 650 kD to 780 kD.
The average molecular weight can be determined by gel permeation chromatography (GPC) and expressed as weight average molecular weight (Mw).

### The topical pharmaceutical compositions

Typically, the present invention provides topical pharmaceutical compositions comprising, based on the total weight of the composition:
(a) 4.0-5.5 wt.% of diclofenac sodium;
(b) 1.5-3.0 wt.% of hyaluronic acid, a salt thereof or mixtures thereof;
(c) 50-60 wt.% of water; and
(d) 30-40 wt.% of a carrier or vehicle other than water.

Preferably, in the topical pharmaceutical compositions according to the invention the total amount of (a) diclofenac sodium is in the range of 4.5-5.5 wt.%, preferably 5.0-5.5 wt.% based on the total weight of the composition.

Preferably, in the topical pharmaceutical compositions according to the invention, the total amount of (b) hyaluronic acid, a salt thereof or mixtures thereof is in the range of 2.0-3.0 wt.%, preferably 2.0-2.5 wt.% based on the total weight of the composition.

Preferably, in the topical pharmaceutical compositions according to the invention, the total amount of (c) water is in the range of 52-60 wt.% based on the total weight of the composition. More preferably, the total amount of (c) water is in the range of 54-60 wt.%, even more preferably 54.7-60 wt%, based on the total weight of the composition.

Preferably, in the topical pharmaceutical compositions according to the invention, the total amount of (d) carrier or vehicle other than water is in the range of 32 to 38 wt.%, based on the total weight of the composition.

Carrier or vehicle refers to carrier material suitable for dermal drug administration and include any such material known in the art, e.g. any liquid, gel, solvent, liquid diluent, solubilizer or the like, which does not interact with other components of the composition in a deleterious manner. Examples of suitable carriers other than water can be found at Martindale - The complete drug reference, 32nd edition, 1999.

Suitable carriers or vehicles other than water according to the invention are petroleum hydrocarbons (mineral oils, paraffins and waxes), animal and vegetable fats and oils, fatty acids, fatty alcohols, non-ionic surfactants, natural waxes, silicones and polyols, or mixtures thereof.

Suitable petroleum hydrocarbons , i.e. mineral oils, paraffins and waxes from petroleum according to the present invention are: hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures which consist mainly of saturated C₁₈-C₃₀ hydrocarbons and smaller amounts of iso-alkanes and cycloalkanes with a molecular weight comprised between 250 and 450 g/mol and, although they are solids at room temperature, they have low melting points, usually comprised between 40ºC and 60ºC), microcrystalline paraffines waxes (which consist of C₄₀-C₅₅ compounds which contain, in addition to normal hydrocarbons, large amounts of iso-alkanes and naphtenes with long alkyl side-chains, the iso-alkanes forming microcrystals, the microcrystalline paraffines waxes having mean molecular weights comprised between 500 and 800 g/mol, being solids at room temperature, and having melting points comprised between 60ºC and 90ºC), or mixtures thereof. Preferred petroleum hydrocarbons are hard paraffin, liquid paraffin, light liquid paraffin, white soft paraffin or mixture thereof, being particularly preferred liquid paraffin, white soft paraffin or mixtures thereof.

Suitable animal or vegetable fats and oils according to the present invention are esters of linear and/or branched, saturated and/or unsaturated alkanecarboxylic acids with a chain length of 1 up to 30 carbon atoms and linear and/or branched, saturated and/or unsaturated alcohols with a chain length of 1 up to 30 carbon atoms; or are esters of aromatic carboxylic acids and linear and/or branched, saturated and/or unsaturated alcohols with a chain length of 1 up to 30 carbon atoms.
These oils can be advantageously selected from the group consisting of isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate, 2-ethylhexyl cocoate, 2-hexyldecyl stearate, 2- ethylhexyl isostearate, 2-octyldodecyl palmitate, cetyl palmitate, oleyl oleate, oleyl erucate, erucyl oleate, erucyl erucate, as well as synthetic, semisynthetic and natural mixtures of such esters, such as jojoba oil (a natural mixture of esters of monounsaturated monocarboxylic acids with a C₁₈-C₂₄ chain with also monounsaturated monoalcohols and with a long C₁₈-C₂₄ chain).
Other suitable oils of the type of esters of saturated alkanecarboxylic acids and alcohols are fatty acid methyl esters, preferably C₆-C₂₄ fatty acid methyl esters from animal and vegetable fats and oils such as cotton, safflower, coconut, rapeseed, linseed, palm, palm kernel, sunflower, olein, olive, olive pomace, castor oil, tallow, soy, tall oil, etc, possibly totally or partially hydrogenated, as well as purified or synthetic fatty such as caproic acid, caprylic acid, capric acid, lauric acid, myristic acid, palmitic acid (cetylic acid), palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or mixtures thereof.

Other suitable animal or vegetable fats and oils according to the present invention are fatty acid triglycerides, specifically triglycerin esters of linear and/or branched, saturated and/or unsaturated alkanecarboxylic acids with a chain length of 6 up to 24 carbon atoms, preferably of 10 up to 18 carbon atoms. The fatty acids esterifying the different positions of glycerin can be different, giving rise to a large amount of possible combinations, including positional combinations. The position of the different fatty acids in natural triglycerides is not random, but rather it depends on the origin of the fat. The triglycerides more simple are those constituted by a sole fatty acid.

Fatty acid triglycerides can be advantageously chosen, for example, from the group consisting of synthetic, semi-synthetic and natural oils, as for example, animal fats and oils such as cow tallow, pig lard, bone oil, aquatic animal fats and oils (fish, such as herring, cod or sardine; cetaceans; etc.); and vegetable fats and oils such as avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and its by-products such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like.

Particularly preferred are vegetable fats and oils as described above.

Suitable fatty acids according to the present invention are C₆-C₂₄ fatty acids from vegetable and animal fats and oils, such as those previously described, such as cotton, safflower, coconut, rapeseed, linseed, palm, palm kernel, sunflower, olein, olive, olive pomace, castor oil, tallow, soy, tall oil, etc, possibly totally or partially hydrogenated, as well as purified or synthetic fatty acids such as caproic acid, caprylic acid, capric acid, lauric acid, myristic acid, palmitic (cetylic) acid, palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or technical grade mixtures thereof. Fatty acids of the lauric, myristic, palmitic, palmitoleic, stearic, isostearic, 2-ethylhexanoic, oleic, ricinoleic, behenic type, or mixtures thereof are preferred, in particular, those from vegetable origin.

Suitable fatty alcohols according to the present invention are C₆-C₂₄ fatty alcohols from vegetable and animal fats and oils such as those previously described, such as cotton, safflower, coconut, rapeseed, linseed, palm, palm kernel, sunflower, olein, olive, olive pomace, castor oil, tallow, soy, tall oil, etc, possibly totally or partially hydrogenated, as well as purified or synthetic fatty alcohols such as caproyl alcohol, capryl alcohol, capric alcohol, lauryl alcohol, myristyl alcohol, palmytil (cetyl) alcohol, palmitoyl alcohol, stearyl alcohol, isostearyl alcohol, 2-octyldodecanol, 2-ethylhexanoyl alcohol, oleyl alcohol, ricinoleyl alcohol, elaidyl alcohol, petroselinic alcohol, linoleyl alcohol, linolenyl alcohol, arachidyl alcohol, gadoleyl alcohol, behenyl alcohol and erucyl alcohol, or technical grade mixtures thereof such as cetostearyl alcohol. Fatty alcohols of the lauryl, myristyl, palmityl, palmitoleyl, stearyl, isostearyl 2-ethylhexanoyl, oleyl, ricinoleyl and behenyl type, or technical grade mixtures thereof such as cetostearyl alcohol are preferred, in particular, those from vegetable origin.

Suitable non-ionic surfactants according to the present invention are acetoglycerides, diacetylated monoglycerides, mono- and di-acetylated monoglycerides, ethylene glycol monostearate, glyceryl behenate, glyceryl mono-oleate, glyceryl monostearate, macrogol cetostearyl ethers, cetomacrogol 1000 (polyoxyethylene glycol 1000 monomethyl ether), polyoxyl 20 cetostearyl ether, macrogol lauryl ethers (such as laureth-2, laureth-4 or laureth-9), macrogol monomethyl ethers (polyethylene glycol monomethyl ethers having nominal average molecular weight in the range of 300 to 10,000, preferably 320 to 4,000, more preferably in the range of 350 to 1,000), macrogol oleyl ethers (polyethylene glycol mono-oleyl ethers), menfegol, mono- and diglycerides, nonoxinois (such as nonoxinol 9, nonoxinol 10 or nonoxinol 11), octoxinols (such as oxtoxinol 9), poloxamers (copolymers of polyoxyethylene and polyoxypropylene, such as poloxamer 188 or poloxamer 188), polyoxyl castor oils (such as poyoxyl 35 castor oil), polyoxyl hydrogenated castor oil (such as polyoxyl 40 hydrogenated castor oil), polyoxyl stearates (such as polyoxyl 8 stearate, polyoxyl 40 stearate or polyoxyl 50 stearate), polysorbates (such as polysorbate 20, polysorbate 40, polysorbate 60, polysorbate 65, polysorbate 80 or polysorbate 85), propylene glycol diacetate, propylene glycol monostearate, quillaia, sorbitan esters (such as sorbitan monolaurate, sorbitan mono-oleate, sorbitan monopalmitate, sorbitan monostearate, sorbitan sesquioleate, sorbitan trioleate, sorbitan tristearate or sucrose esters. Particularly preferred non-ionic surfactants are cetomacrogol 1000 (polyoxyethylene glycol 1000 monomethyl ether), polyoxyl 20 cetostearyl ether, macrogol lauryl ethers (such as laureth-2, laureth-4 or laureth-9), macrogol monomethyl ethers (polyethylene glycol monomethyl ethers having nominal average molecular weight in the range of 300 to 10,000, preferably 350 to 4,000) and polysorbates. Even more preferred non-ionic surfactants are macrogol monomethyl ethers (polyethylene glycol monomethyl ethers having nominal average molecular weight in the range of 300 to 10,000, preferably 320 to 4,000, more preferably in the range of 350 to 1,000)

Suitable natural waxes according to the present invention are the candelilla wax, carnauba wax, Japan wax, esparto wax, cork wax, guaruma wax, rice wax, sugar cane wax, ouricury wax, montan wax, beeswax, shellac wax, espermaceti, wool lanolin (wax), uropygial fat wax, ceresin waxes, peat waxes, ozokerite, as well as chemically modified waxes (hard waxes) for example, montan wax esters, waxes obtained by the Fischer-Tropsch process, hydrogenated jojoba waxes and synthetic waxes.

Silicones suitable according to the present invention are cyclic and/or linear silicones, which can be found as monomers generally characterized by structural elements such as: 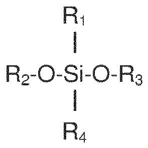 where the silicon atoms can be substituted by alkyl or aryl radicals equal or different, represented here generally by R₁-R₄ groups.

Linear silicones with siloxane units suitable according to the present invention are generally characterized by structural elements such as: 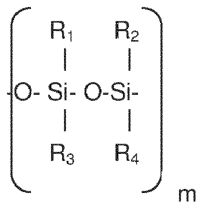 where the silicon atoms can be substituted by alkyl or aryl radicals equal or different, are represented here in general by R₁-R₄ groups (meaning the number of different radicals is not necessarily limited to 4), m can take values from 2 to 200.000.

Cyclic silicones suitable according to the present invention are generally characterized by structural elements such as: 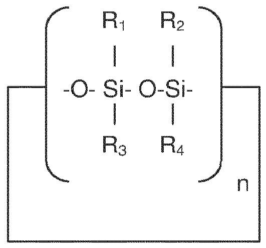 where the silicon atoms can be substituted by alkyl or aryl radicals equal or different, represented here generally by R₁-R₄ groups (meaning the number of different radicals is not necessarily limited to 4), n can take values of 3/2 to 20. Fractional values of n indicate that it may be odd numbers of siloxane groups present in the ring.

Specific examples include a cyclic methyl siloxane having the formula [(CH₃)₂SiO]ₓ in which x is 3-6, or short chain linear methyl siloxanes having the formula ((CH₃)₂SiO[(CH₃)₂SiO]_{y}Si(CH₃)₃ in which y is 0-5.

Some suitable cyclic methyl siloxanes are hexamethylcyclotrisilaxanes (D3), a solid with a boiling point of 134ºC and the formula [(Me₂)SiO]₃; octamethylcyclotetrasiloxane (D4) with a boiling point of 176ºC, a viscosity of 2.3 mm²/s, and the formula [(Me₂)SiO]₄; decamethylcyclopentasiloxane (D5) (cyclomethicone) with a boiling point of 210ºC, a viscosity of 3.87 mm²/s, and the formula [(Me₂)SiO]₅; and dodecamethylcyclohexasiloxane (D6) with a boiling point of 245ºC, a viscosity of 6.62 mm²/s and the formula [(Me₂)SiO]₆.

Some suitable short linear methyl siloxane are hexamethyldisiloxane (MM) with a boiling point of 100ºC, viscosity of 0-65 mm²/s, and formula Me₃SiOMe₃; octamethyltrisiloxane (MDM) with a boiling point of 152ºC, viscosity of 1.04 mm²/s, and formula Me₃SiOMe₂SiOSiMe₃; decamethyltetrasiloxane (MD2M) with a boiling point of 194ºC, viscosity of 1.53 mm²/s, and formula Me₃SiO(MeSiO)₂SiMe₃; dodecamethylpentasiloxane (MD3M) with a boiling point of 229ºC, viscosity of 2.06 mm²/s, and formula Me₃SiO(Me₂SiO)₃SiMe₃; tetradecamethylhexasiloxane (MD4M) with a boiling point of 245ºC, viscosity of 2.63 mm²/s, and formula Me₃SiO(Me₂SiO)₄SiMe₃; and hexadecamethylheptasiloxane (MD5M) with a boiling point of 270ºC, viscosity of 3.24 mm²/s, and formula Me₃SiO(Me₂SiO)₅SiMe₃.

Furthermore, long chain linear siloxanes such as phenyltrimethicone, bis(phenylpropyl)dimethicone, dimethicone, dimethiconol, cyclomethicone (octametilciclotetrasiloxane), hexamethylcyclotrisiloxane, poly(dimethylsiloxane), cetyldimethicone and behenoxy dimethicone are also included.

In addition, mixtures of cyclomethicone and isotridecyl isononanoate and of cyclomethicone and 2-ethylhexyl isostearate are also suitable silicones according to the invention.

Suitable polyols according to the present invention are preferably water-soluble polyols such as polyhydric alcohols with two or more hydroxyl groups in their molecule. Specific examples can include ethylene glycol, propylene glycol, 1,3- butylene glycol, 1,4-butylene glycol, hexylene glycol, dipropylene glycol, polyethylene glycol with average molecular weights by weight ranging between 100 and 1000, glucose, fructose, galactose, mannose, ribose, erythrose, maltose, maltitose, maltotriose, sucrose, xylitol, sorbitol, threitol, erythritol, glycerol, polyglycerol and starch alcohols. Preferred polyols are ethylene glycol, propylene glycol, 1,3-butylene glycol, 1,4-butylene glycol, hexylene glycol, dipropylene glycol, polyethylene glycol with average molecular weights by weight ranging between 100 and 1000, glycerol, polyglycerol, and mixtures thereof.

Preferred carrier or vehicles according to the invention are non-ionic surfactants selected from cetomacrogol 1000 (polyoxyethylene glycol 1000 monomethyl ether), polyoxyl 20 cetostearyl ether, macrogol lauryl ethers (such as laureth-2, laureth-4 or laureth-9), macrogol monomethyl ethers (polyethylene glycol monomethyl ethers having nominal average molecular weight in the range of 300 to 10,000, preferably 320 to 4,000, even more preferably 350 to 1,000) and polysorbates. Even more preferred carrier or vehicles are macrogol monomethyl ethers (polyethylene glycol monomethyl ethers having nominal average molecular weight in the range of 300 to 10,000, preferably 320 to 4,000, even more preferably 350 to 1,000)

Preferred topical pharmaceutical compositions of the invention comprise, based on the total weight of the composition:
(a) 4.5-5.5 wit.% of diclofenac sodium:
(b) 2.0-3.0 wit.% of hyaluronic acid, a salt thereof or mixtures thereof;
(c) 52-60 wit.% of water; and
(d) 32 to 38 wt.% of a carrier or vehicle.

The topical pharmaceutical compositions according to the invention may optionally further comprise (e) a preservative agent.

Typically, in the topical pharmaceutical compositions according to the invention the preservative agent is selected from the group consisting of alkylparabens, particularly methylparaben, propylparaben and butylparaben; benzalkonium chloride; benzethonium chloride; benzoic acid; benzyl alcohol; sodium benzoate; bronopol; butylated hydroxy toluene; butylated hydroxyanisole; cetrimide; chlorobutanol; chlorocresol; chlorhexidine; dehydroacetic acid; ethylenediamine tetraacetic acid; paraben esters; phenylethylalcohol; phenol; phenoxyethanol; sorbic acid; potassium sorbate; and mixtures thereof. The amount of preservative generally utilized will vary depending upon the preservative selected.

Preferred preservative agents according to the invention are benzyl alcohol, phenylethylalcohol and phenoxyethanol. Benzyl alcohol is particularly preferred. Preferably, the preservative agent (e) is used in a concentration ranging from 0.5 to 2.0 wit.%, preferably from 0.8 to 1.5 wit.% based on the total amount of the composition.

In a particularly preferred embodiment, the topical pharmaceutical compositions of the invention comprise, based on the total weight of the composition:
(a) 4.5-5.5 wt.% of diclofenac sodium;
(b) 2.0-3.0 wt.% of hyaluronic acid, a salt thereof or mixtures thereof;
(c) 52-60 wt.% of water;
(d) 32 to 38 wt.% of a carrier or vehicle, and
(e) 0.5 to 2.0 wt.% of a preservative.

In another particularly preferred embodiment, the topical pharmaceutical compositions of the invention comprise, based on the total weight of the composition:
(a) 5.0-5.5 wit.% of diclofenac sodium;
(b) 2.0-2.5 wit.% of sodium hyaluronate;
(c) 52-60 wit.% of water;
(d) 32 to 38 wt.% of a carrier or vehicle, and
(e) 0.8 to 1.5 wit.% of a preservative.

In a further particularly preferred embodiment, the topical pharmaceutical compositions of the invention comprise, based on the total weight of the composition:
(a) 5.0-5.5 wt.% of diclofenac sodium;
(b) 2.0-2.5 wit.% of sodium hyaluronate;
(c) 52-60 wt.%, preferably 54-60 wit.%, more preferably 54.7-60 wit% of water;
(d) 32 to 38 wt.% of polyethylene glycol monomethyl ether having nominal average molecular weight in the range of 300 to 10,000; and
(e) 0.8 to 1.5 wt.% of benzyl alcohol.

The topical pharmaceutical compositions according to the invention may optionally further comprise other well-known pharmaceutically and/or cosmetically acceptable additives, such as, e.g. anti-irritants, antioxidants, buffering agents (pH adjusting agents), chelating agents, emollients, penetration enhancing agents, solubilizing agents, thickening agents, wetting agents, and the like, or mixtures thereof.

Examples of suitable anti-irritants are aloe vera, chamomile, alpha-bisabolol, cola nitida extract, green tea extract, tea tree oil, licoric extract, batyl alcohol (α-octadecyl glyceryl ether), selachyl alcohol (α-9-octadecenyl glyceryl ether), chimyl alcohol (α-hexadecyl glyceryl ether), panthenol, allantoin, caffeine or other xanthines, glycyrrhizic acid and derivatives thereof, and mixtures thereof.

Antioxidants used can be any antioxidants which are suitable or customary for cosmetic and/or dermatological applications. Suitable antioxidants are advantageously selected from the group consisting of amino acids (for example glycine, histidine, tyrosine, tryptophan) and derivatives thereof, imidazoles (e.g. urocanic acid) and derivatives thereof, peptides such as D,L-carnosine, D-carnosine, L-carnosine and derivatives thereof (e.g. anserine), carotenoids, carotenes (e.g. α-carotene, β-carotene, lycopene) and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts) and sulphoximine compounds (e.g. buthionine sulphoximines, homocysteine sulphoximine, buthionine sulphones, penta-, hexa-, heptathionine sulphoximine) in very small tolerated doses (e.g. pmol to µmol/kg), also (metal) chelating agents (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), and coniferylbenzoate of benzoin, rutinic acid and derivatives thereof, ferulic acid and derivatives thereof, butylated hydroxytoluene, butylated hydroxyanisole, nordihydroguaiac resin acid, nordihydroguaiaretic acid, trihydroxybutyrophenone, uric acid and derivatives thereof, mannose and derivatives thereof, zinc and derivatives thereof (e.g. ZnO, ZnSO₄), selenium and derivatives thereof (e.g. selenium methionine), stilbenes and derivatives thereof (e.g. stilbene oxide, trans-stilbene oxide) and the derivatives (salts, esters, ethers, sugars, nucleotides, nucleosides, peptides and lipids) of said active ingredients which are suitable according to the invention.

Any pharmaceutically acceptable buffering agents to adjust the pH of the aqueous liquid pharmaceutical compositions according to the invention to be within the acceptable range for topical administration, preferably in the range of 3.0 to 6.0, more preferably in the range of 3.5 to 5, can be used. For example the inclusion in the composition of a pharmaceutically acceptable acid such as acetic, citric, fumaric, phosphoric, hydrochloric, lactic or nitric acids or the like, or a mixture thereof. It will also be understood that certain compositions of the invention can have a pH in the desired range without inclusion of a pH adjusting agent specifically for that purpose. Typically, however, an acidic buffer system is present in the composition to achieve the desired pH. An acidic buffer system comprises an acidulant and a buffering agent. Suitable acidulants will be known to those of skill in the art and illustratively include acetic, citric, fumaric, hydrochloric, phosphoric, lactic and nitric acids and the like, and mixtures thereof. Suitable buffering agents will likewise be known to those of skill in the art and illustratively include potassium metaphosphate, potassium phosphate, sodium phosphate, sodium acetate, sodium citrate and the like, and mixtures thereof.

Suitable emollients, which can be used in the composition of the present invention include, for example, dodecane, squalane, cholesterol, isohexadecane, isononyl isononanoate, PPG Ethers, petrolatum, lanolin, safflower oil, castor oil, coconut oil, cottonseed oil, palm kernel oil, palm oil, peanut oil, soybean oil, polyol carboxylic acid esters, derivatives thereof, and the like, and combinations thereof.

Examples of suitable penetration enhancing agents can include, e.g., dimethylsulfoxide (DMSO), N-methyl pyrrolidine, dimethyl formamide (DMF), allantoin, urazole, N,N-dimethylacetamide (DMA), decylmethylsulfoxide, polyethylene glycol monolaurate, propylene glycol, propylene glycol monolaurate, glycerol monolaurate, lecithin, the 1-substituted azacycloheptan-2-ones, particularly 1-n-dodecylcyclazacycloheptan-2-one, alcohols, glycerin, hyaluronic acid, transcutol, and the like, and combinations thereof. Certain oil components (e.g., certain vegetable oils such as, e.g., safflower oil, cottonseed oil and corn oil) also can exhibit penetration enhancing properties.

Examples of solubilizing agents are, for example, nonionic surfactants from at least one of the following groups: products of the addition of 1 to 30 moles of ethylene oxide and/or 0 to 5 moles of propylene oxide onto linear C₈-C₂₂ fatty alcohols, C₁₂-C₂₂ fatty acids and alkyl phenols containing 8 to 15 carbons in the alkyl group; alkyl and/or alkenyl oligoglycosides containing 8 to 22 carbons in the alkyl group and ethoxylated analogs thereof; addition products of 1 to 15 moles of ethylene oxide with castor oil and/or hydrogenated castor oil; addition products of 15 to 60 moles of ethylene oxide with castor oil and/or hydrogenated castor oil; partial esters of glycerol and/or sorbitan with unsaturated or saturated, linear or branched fatty acids containing 12 to 22 carbons and/or hydroxycarboxylic acids containing 3 to 18 carbon atoms and addition products thereof with 1 to 30 moles of ethylene oxide; mixtures of alkoxylated glycerides and alkoxylated glycerine, partial esters of polyglycerol (average degree of selfcondensation 2 to 8), polyethylene glycol (weight average molecular weight 400 to 5000), trimethylolpropane, pentaerythritol, sugar alcohols (for example sorbitol), alkyl glucosides (for example methyl glucoside, butyl glucoside, lauryl glucoside) and polyglucosides (for example cellulose) with saturated and/or unsaturated, linear or branched fatty acids containing 12 to 22 carbons and/or hydroxycarboxylic acids containing 3 to 18 carbons and addition products thereof with 1 to 30 moles of ethylene oxide; mixed esters of pentaerythritol, fatty acids, citric acid and fatty alcohol and/or mixed esters of fatty acids containing 6 to 22 carbons, methyl glucose and polyols, preferably glycerol or polyglycerol; mono-, di- and trialkyl phosphates and mono-, di-and/or tri-PEG-alkyl phosphates and salts thereof; block copolymers, for example Polyethyleneglycol-30 Dipolyhydroxystearate; polymer emulsifiers; polyalkylene glycols and alkyl glyceryl ethers. Particularly preferred solubilizing agents are products of the addition of 1 to 30 moles of ethylene oxide and/or 0 to 5 moles of propylene oxide onto linear C₈-C₂₂ fatty alcohols such as lauryl, myristyl, cetyl (palmityl), stearyl, oleyl, and ricinoleyl alcohols, or technical grade mixtures thereof such as cetostearyl alcohol or palmitoleyl alcohol.

A thickening agent or viscosity-enhancing agent can be included to generally thicken the liquid pharmaceutical compositions. While any suitable thickening agent can be included in the compositions of the present invention, a preferred thickening agent, when used, includes one or more of acacia, alginic acid bentonite, carbomer, carboxymethylcellulose calcium or sodium, cetostearyl alcohol, methyl cellulose, ethylcellulose, glycerin, gelatin guar gum, hydroxyethylcellulose, hydroxypropylcellulose, hydroxypropylmethylcellulose, maltodextrin, polyvinyl alcohol, povidone, propylene carbonate, propylene glycol alginate, sodium alginate, sodium starch glycolate, starch tragacanth, and xanthan gum, and any combination thereof. More preferred thickening agents are glycerin, hydroxypropylmethylcellulose, and xanthan gum, and any combination thereof.

Examples of wetting agents (chemical substances that increase the spreading and penetrating properties of a liquid by lowering its surface tension) include one or more cationic surfactants, such as benzalkonium chloride; non-ionic surfactants such as polyoxyethylene and polyoxypropylene block copolymers; polyoxyethylene fatty acid glycerides and oils (such as polyoxyethylene (6) caprylic/capric mono- and diglycerides), polyoxyethylene (40) hydrogenated castor oil; polyoxyethylene sorbitan esters, such as polysorbate 20 and polysorbate 80; propylene glycol fatty acid esters, such as propylene glycol laureate; glyceryl fatty acid esters, such as glyceryl monostearate; sorbitan esters, such as sorbitan monolaurate, sorbitan monooleate, sorbitan monopalmitate and sorbitan monostearate; glyceryl fatty acid esters, for example glyceryl monostearate; anionic surfactants such as sodium lauryl sulphate, sodium lauryl ether sulphate; or fatty acids and salts thereof, such as oleic acid, sodium oleate and triethanolamine oleate.

In a particularly preferred embodiment, the topical pharmaceutical compositions of the invention comprise, based on the total weight of the composition:
(a) 5.0 wt.% of diclofenac sodium;
(b) 2.5 wit.% of sodium hyaluronate;
(c) 55-60 wt.% of water;
(d) 32 to 35 wit.% of polyethylene glycol monomethyl ether having nominal average molecular weight in the range of 300 to 10,000; and
(e) 0.8 to 1.5 wt.% of benzyl alcohol.

In a further particularly preferred embodiment, the topical pharmaceutical compositions of the invention comprise, based on the total weight of the composition:
(a) 5.5 wt.% of diclofenac sodium;
(b) 2.0 wt.% of sodium hyaluronate;
(c) 54-58 wt.%, preferably 54.7-58 wt.% of water;
(d) 35 to 38 wt.% of polyethylene glycol monomethyl ether having nominal average molecular weight in the range of 300 to 10,000; and
(e) 0.8 to 1.5 wt.% of benzyl alcohol.

The pH value of the topical pharmaceutical compositions according to the invention is typically within the acceptable range for topical administration, and is preferably in the range of 3.0 to 9.0, more preferably in the range of 4.5 to 8.5, even more preferably in the range of 6.0 to 8.0.

The topical pharmaceutical composition of the invention is preferably formulated in the form of a gel.

As used herein, gel is a homogeneous, clear, semi-solid preparation consisting of a liquid phase within a three-dimensional polymeric matrix with physical or sometimes chemical cross-linkage by means of suitable gelling agents (International Pharmacopoeia, 4th edition).

The viscosity of the topical pharmaceutical compositions according to the invention is typically in the range of 2,000 to 15,000 mPa.s, preferably in the range of 2,500 to 12,000 mPa.s, more preferably in the range of 7,000 to 11,000 mPa.s measured at 20°C using a DIN-Rotations Rheometer (Paar Physica); Measuring System Z 3 DIN ; D=57 1/s.

The invention further relates to a topical pharmaceutical composition as defined above for use in the treatment or prevention of skin disorders or diseases selected from a) epidermal and follicular tumors (such as basal cell carcinoma (BCC), squamous cell carcinoma (SCC), actinic keratosis (AK), Bowen's disease, erythroplasia of Queyrat, leukoplakia, oral florid papillomatosis, verrucous carcinoma or keratoacanthoma); b) sebaceous gland tumors (such as sebaceous carcinoma); c) sweat gland tumors (such as mammary Paget's disease, extramammary Paget's disease, eccrine porocarcinoma, microcystic adnexal carcinoma (MAC) or mucinous carcinoma of the skin); d) nervous system tumors (such as Merkel cell carcinoma (MCC)); e) mesenchymal tumors (such as dermatofibrosarcoma protuberans (DFSP), malignant fibrous histiocytoma (MFH), atypical fibroxanthoma, epithelioid sarcoma, synovial sarcoma, liposarcoma, angiosarcoma, Kaposi's sarcoma, cutaneous lymphoma or multiple myeloma); f) metastatic carcinoma of the skin; g) skin melanoma; h) lamellar ichthyosis; i) acne; j) hirsutism; k) androgenetic alopecia; I) pruritus (itch); or m) scar.
Preferably, the skin disease or disorder is selected from the group consisting of basal cell carcinoma (BCC), squamous cell carcinoma (SCC), actinic keratosis (AK), Bowen's disease, erythroplasia of Queyrat, leukoplakia, oral florid papillomatosis, verrucous carcinoma or keratoacanthoma.
In a preferred embodiment, the skin disease or disorder is selected from the group consisting of basal cell carcinoma (BCC), squamous cell carcinoma (SCC) and actinic keratosis (AK); preferably the skin disease or disorder is actinic keratosis (AK).

The invention further relates to the use of a topical pharmaceutical composition as defined above for the manufacture of a medicament for the treatment or prevention of skin disorders or diseases as defined above.

The invention further relates to a method for treating a subject afflicted skin disorders or diseases as defined above, which comprises applying to the affected area of skin of said subject an effective amount of a topical pharmaceutical composition as defined above.

The method of using the topical pharmaceutical composition of the invention is by applying it to completely cover the affected area, forming an occlusive barrier. The usual frequency of application is once or twice daily. The amount needed depends upon the size of the lesion site.

Typically, the affected area of the skin affected by the disease or disorder is selected from the group consisting of the face, ears, scalp, neck, forearms, back, legs, arms and hands.

The following examples are given in order to provide a person skilled in the art with a sufficiently clear and complete explanation of the present invention, but should not be considered as limiting of the essential aspects of its subject, as set out in the preceding portions of this description.

### EXAMPLES

### Example 1

In a manner known er se in the art (see US 5792753), the compositions indicated in Table 1 were prepared:

Ex. 1 and Ex. 2 are compositions of the invention and C1-C3 are comparative experiments.

**Table 1 - Topical pharmaceutical compositions**

| Ingredients (wt.%) | Ex. 1 | Ex. 2 | C1 | C2 | C3 |
|---|---|---|---|---|---|
| Diclofenac sodium | 5.0 | 5.5 | 7.5 | 6.0 | 6.0 |
| Sodium hyaluronate¹ | 2.5 | 2.0 | 2.5 | 2.5 | 2.0 |
| Polyethylene glycol monomethyl ether 350 | 33.5 | 36.7 | 50.0 | 40 | 40 |
| Benzyl alcohol | 1.0 | 1.0 | 1.0 | 1.0 | 1.0 |
| Purified Water | up to 100 % | up to 100 % | up to 100 % | up to 100 % | up to 100 % |
| Is a gel composition obtained? | Yes | Yes | No | No | No |

| | | | | | |
|---|---|---|---|---|---|
| ¹Having an average molecular weight 600-800 kD | | | | | |

While Ex. 1 and Ex. 2 are clear transparent, viscous gel compositions, in comparative experiments C1-C3 sodium hyaluronate swells and no viscous gel compositions are formed.
From the experimental results it can be concluded that only when the ingredients of the composition are within the claimed ranges, gel compositions are obtained.

### Example 2

The viscosity of Example 1 was measured at 20°C using a DIN-Rotations Rheometer (Paar Physica); Measuring System Z 3 DIN; D= 57 1/s.

Additionally, the viscosity of formulation 3 of US 5792753 (gel formulation comprising diclofenac sodium 3% gel and 2.5% sodium hyaluronate - comparative example C4) was determined using the same measuring conditions.

The results are indicated in Table 2.

**Table 2 - Viscosity values**

| Ex. 1 | C4 |
|---|---|
| aprox. 8,000 mPa.s | aprox. 8,000 mPa.s |

From the experimental results it can be concluded that the composition of Example 1 (according to the invention) can be applied as easily as compositions having lower concentrations of diclofenac sodium. Thus, the composition of the invention can be applied in an acceptable way by the general patient population.

Modifications, which do not affect, alter, change or modify the essential aspects of the compounds, combinations or pharmaceutical compositions described, are included within the scope of the present invention.

## Claims

1. A topical pharmaceutical composition comprising, based on the total weight of the composition:
(a) 4.0-5.5 wt.% of diclofenac sodium;
(b) 1.5-3.0 wt.% of hyaluronic acid, a salt thereof or mixtures thereof;
(c) 50-60 wt.% of water; and
(d) 30-40 wt.% of a carrier or vehicle other than water.

2. A composition according to claim 1, wherein the total amount of (a) diclofenac sodium is in the range of 4.5-5.5 wt.%, preferably 5.0-5.5 wt.% based on the total weight of the composition.

3. A composition according to claim 1 or 2, wherein the total amount of (b) hyaluronic acid, a salt thereof or mixtures thereof is in the range of 2.0-3.0 wt.%, preferably 2.0-2.5 wt.% based on the total weight of the composition.

4. A composition according to any one of the preceding claims, wherein the total amount of (c) water is in the range of 52-60 wt.% based on the total weight of the composition.

5. A composition according to any one of the preceding claims, wherein the total amount of (d) carrier or vehicle is in the range of 32 to 38 wt.%, based on the total weight of the composition.

6. A composition according to claim 1 comprising based on the total weight of the composition:
(a) 4.5-5.5 wt.% of diclofenac sodium:
(b) 2.0-3.0 wt.% of hyaluronic acid, a salt thereof or mixtures therof;
(c) 52-60 wt.% of water; and
(d) 32 to 38 wt.% of a carrier or vehicle.

7. A composition according to any one of the preceding claims, wherein component (b) is selected from hyaluronic acid, sodium hyaluronate of mixtures thereof.

8. A composition according to any one of the preceding claims, wherein the composition further comprises (e) a preservative agent.

9. A composition according to claim 8, wherein the preservative agent (e) is selected from the group consisting of alkylparabens; benzalkonium chloride; benzethonium chloride; benzoic acid; benzyl alcohol; sodium benzoate; bronopol; butylated hydroxy toluene; butylated hydroxyanisole; cetrimide; chlorobutanol; chlorocresol; chlorhexidine; dehydroacetic acid; ethylenediamine tetraacetic acid; paraben esters; phenylethylalcohol; phenol; phenoxyethanol; sorbic acid; potassium sorbate; and mixtures thereof.

10. A composition according to any one of the preceding claims, wherein the composition further comprises anti-irritants agents, antioxidants agents, buffering agents, chelating agents, emollients, penetration enhancing agents, solubilizing agents, thickening agents, wetting agents or mixtures thereof.

11. A composition according to any one of the preceding claims, wherein the composition has a pH value in the range of 3.0 to 9.0.

12. A composition according to any one of the preceding claims, wherein the composition is formulated in the form of a gel.

13. A composition as defined in any one of claims 1 to 12, for use in the treatment or prevention of skin disorders or diseases selected from a) epidermal and follicular tumors (such as basal cell carcinoma (BCC), squamous cell carcinoma (SCC), actinic keratosis (AK), Bowen's disease, erythroplasia of Queyrat, leukoplakia, oral florid papillomatosis, verrucous carcinoma or keratoacanthoma); b) sebaceous gland tumors (such as sebaceous carcinoma); c) sweat gland tumors (such as mammary Paget's disease, extramammary Paget's disease, eccrine porocarcinoma, microcystic adnexal carcinoma (MAC) or mucinous carcinoma of the skin); d) nervous system tumors (such as Merkel cell carcinoma (MCC)); e) mesenchymal tumors (such as dermatofibrosarcoma protuberans (DFSP), malignant fibrous histiocytoma (MFH), atypical fibroxanthoma, epithelioid sarcoma, synovial sarcoma, liposarcoma, angiosarcoma, Kaposi's sarcoma, cutaneous lymphoma or multiple myeloma); f) metastatic carcinoma of the skin; g) skin melanoma; h) lamellar ichthyosis; i) acne; j) hirsutism; k) androgenetic alopecia; I) pruritus (itch); or m) scar.

14. Use of a composition as defined in any one of claims 1 to 12, for the manufacture of a medicament for the treatment or prevention of skin disorders or diseases as defined in claim 13.

15. Method for treating a subject afflicted with skin disorders or diseases as defined in claim 13, which comprises applying to the affected area of skin of said subject an effective amount of a composition as defined in any one of claims 1 to 12.
